# EUROPEAN PATENT SPECIFICATION

(11) **EP 2 176 282 B1**
(45) Date of publication and mention of the grant of the patent: **22.08.2012**
(21) Application number: 08775031.1
(22) Date of filing: 11.07.2008
(51) Int. Cl.: C07J 1/00, A61K 31/565, A61P 5/30

(54) **8-BETA-SUBSTITUTED 16.ALPHA.-FLUORO-ESTRATRIENES AS SELECTIVELY ACTIVE ESTROGENS**
8-BETA-SUBSTITUIERTE 16.ALPHA.-FLUORO-ÖSTRATRIENE ALS SELEKTIV AKTIVE ÖSTROGENE
16.ALPHA.-FLUORO-ESTRATRIÈNES À SUBSTITUTION 8-BÊTA SERVANT D' ESTROGÈNES SÉLECTIVEMENT ACTIFS

(30) Priority: 12.07.2007 EP 07075600
(43) Date of publication of application: 21.04.2010
(73) Proprietor: Bayer Pharma Aktiengesellschaft, 13353 Berlin (DE)
(72) Inventor: PETERS, Olaf, 99891 Tabarz (DE); BRAEUER, Nico, 14612 Falkensee (DE); THIEME, Ina, 07616 Graitschen b. Bürgel (DE); PRELLE, Katja, 13465 Berlin (DE); MUHN, Hans-Peter, 13465 Berlin (DE); FRITZEMEIER, Karl-Heinrich, 13505 Berlin (DE)
(86) International application number: PCT/EP2008/059115
(87) International publication number: WO 2009/007454

(56) References cited:
- WO-A-01/77139
- YOO JEONGSOO ET AL: "Synthesis of an estrogen receptor beta-selective radioligand: 5-[F-18]Fluoro-(2R*,3S*)-2,3-bis(4-hydroxy phenyl)pentanenitrile and comparison of in vivo distribution with 16 alpha-[F-18]Fluoro-17 beta-estradiol" JOURNAL OF MEDICINAL CHEMISTRY, vol. 48, no. 20, October 2005 (2005-10), pages 6366-6378, XP002458224 ISSN: 0022-2623
- VANBROCKLIN HENRY F ET AL: "Fluorine-18 16-beta-( intg luoro)estrogens: Systematic investigation of a new series of fluorine-18-labeled estrogens as potential imaging agents for estrogen-receptor-positive breast tumors" JOURNAL OF MEDICINAL CHEMISTRY, vol. 36, no. 11, 1993, pages 1619-1629, XP002458225 ISSN: 0022-2623

## Description

### Field of the Invention

This invention relates to new 8β-substituted estra-1,3,5(10)-triene derivatives of general formula 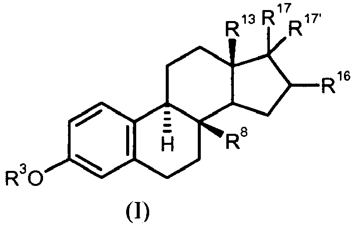 in which radicals R³, R⁸, R¹³, R¹⁶ as well as R¹⁷ and R^{17'}, independently of one another, have the following meaning:
R³ means a hydrogen atom or a group R¹⁸, in which
   R¹⁸ means a straight-chain or branched-chain, saturated or unsaturated
      C₁-C₆-alkyl radical, a trifluoromethyl group,
      an aryl, heteroaryl or aralkyl radical optionally substituted with at least one radical independently chosen from a methyl, ethyl, trifluoromethyl,
      pentafluoroethyl, trifluoromethylthio, methoxy, ethoxy, nitro, cyano, halo-,
      hydroxy, amino,
      mono(C₁-C₈-alkyl) or di(C₁-C₈-alkyl)amino whereby both alkyl groups are identical or different, di(aralkyl)amino whereby both aralkyl groups are identical or different, carboxyl, carboxyalkoxy, C₁-C₂₀-acyl or C₁-C₂₀-acyloxy as substituents;
      an acyl radical -C(=O)R¹⁹, in which R¹⁹ is a straight-chain or branched-chain of a C₁-C₁₀ -alkyl radical that is saturated or unsaturated in up to three places and is partially or completely halogenated, or
   R¹⁸ means a group R²⁰SO₂, in which
      R²⁰ is an R²¹R²²N group, whereby R²¹ and R²², independently of one another, mean a hydrogen atom, a C₁-C₅-alkyl radical, a group -C(=O)R²³, in which R²³ means a unsubstituted or substituted, straight-chain or branched-chain C₁-C₁₀ -alkyl radical with that is saturated or unsaturated in up to three places and is partially or completely halogenated, a cyclopropyl, cyclobutyl, cyclopentyl, cyclohexyl or cycloheptyl group, a C₄-C₁₅-cycloalkylalkyl radical with 3 to 7 carbon atoms in the cycloalkyl portion and with an alkyl portion of up to 8 carbon atoms or an aryl, heteroaryl or aralkyl radical, optionally substituted, with at least one radical independently chosen from a methyl, ethyl, trifluoromethyl, pentafluroethyl, trifluoromethylthio, methoxy, ethoxy, nitro, cyano, halo-, hydroxy, amino, mono(C₁-C₈-alkyl) or di(C₁-C₈-alkyl) amino, whereby both alkyl groups are identical or different, di(aralkyl)amino, whereby both aralkyl groups are identical or different, carboxyl, carboxyalkoxy, C₁-C₂₀-acyl or C₁-C₂₀-acyloxy as substituents; or,
         together with the N atom, a polymethylenimino radical with 4 to 6 carbon atoms or a morpholino radical,
   R⁸ is a straight-chain or branched-chain alkenyl or alkinyl radical with 2 to 6 carbon atoms, which optionally can be partially or completely fluorinated,
   R¹³ is a methyl group or an ethyl group,
R¹⁶ is a fluorine atom in the ∝ position,
R¹⁷ and R^{17'}, in each case independently of one another, are a hydrogen atom and a hydroxy group; or
   a hydrogen atom and a group R¹⁸O-, R²⁰SO₂- or OC(O)R²³ with R¹⁸, R²⁰ and R²³ in each case in the meaning that is indicated under R³.

This invention further relates to the use of the new 8β-substituted estra-1,3,5(10)-triene as pharmaceutical active ingredients, which have in vitro a higher affinity to estrogen receptor preparations from rat prostates than to estrogen receptor preparations from rat uteri and in vivo a preferential action in the ovary in comparison to the uterus, their production, their therapeutic use and pharmaceutical dispensing forms that contain the new compounds.

The 8β-substituted estra-1,3,5(10)-triene derivatives of the present invention are new, steroidal, estrogen receptor subtype -selective estratriene with improved potency and metabolic stability.

### Background of the Invention

The efficiency of estrogens in the treatment of hormone-deficiency-induced symptoms such as hot flashes, atrophy of estrogen target organs and incontinence, as well as the successful use of estrogen therapies for prevention of bone mass loss in peri- and postmenopausal women, is well documented and generally accepted (Grady et al. 1992, Ann Intern Med 117: 1016-1037). It is also well documented that estrogen replacement therapy in postmenopausal women or in women with ovarian dysfunction that is caused in some other way reduces the risk of cardiovascular diseases compared to women who are not treated with estrogen (Grady et al., loc. cit.).

In conventional estrogen or hormone replacement therapy (= HRT), natural estrogens, such as estradiol, and conjugated estrogens that consist of equine urine are used either by themselves or in combination with a gestagen. Instead of the natural estrogens, derivatives that are obtained by esterification, such as, e.g., 17β-estradiol-valerate, can also be used.

Because of the stimulating action of the estrogens that are used on the endometrium, which results in an increase of the risk of endometrial carcinoma (Harlap, S. 1992, Am J Obstet Gynecol 166: 1986-1992), estrogen/gestagen combination preparations are preferably used in hormone replacement therapy. The gestagenic component in the estrogen/gestagen combination avoids hypertrophy of the endometrium, but the occurrence of undesirable intracyclic menstrual bleeding is also linked to the gestagen-containing combination.

Selective estrogens represent a more recent alternative to the estrogen/gestagen combination preparations. Up until now, selective estrogens have been defined as those compounds that have an estrogen-like effect on the brain, bones and vascular system, owing to their antiuterotropic (i.e., antiestrogenic) partial action, but they do not have a proliferative effect on the endometrium.

A class of substances that partially meet the desired profile of a selective estrogen is the so-called "Selective Estrogen Receptor Modulators" (SERM) (R. F. Kauffman, H. U. Bryant 1995, DNAP 8 (9): 531-539). In this case, these are partial agonists of estrogen receptor subtype "ERα." This substance type is ineffective, however, with respect to the therapy of acute postmenopausal symptoms, such as, e.g., hot flashes. As an example of a SERM, the raloxifene that was recently introduced for the indication of osteoporosis can be mentioned.

For the treatment of fertility disorders of women, frequently caused by ovarian dysfunction that is caused by surgery, medication, etc., new possible therapies are also opened up by the use of new selective estrogens. The in-vitro fertility treatment is a process that has been established for more than 20 years. Numerous methods for treating ovarian-induced infertility with exogenic gonadotropins are known. By administration of gonadotropins such as FSH (FSH = follicle-stimulating hormone), a stimulation of the ovaries, which is to make possible a healthy follicular maturation, is to be produced.

The follicle is the functional unit of the ovary and has two purposes: it accommodates the oocytes and provides for the latter the possibility for growth and for maturation. Folliculogenesis comprises the development of an ovarian follicle from a primordial stage to a continuously increasing antral follicle, which represents the last stage before ovulation. Only an optimally developed antral follicle can release a mature oocyte by ovulation.

Patients with ovarian-induced infertility (PCOS = syndrome of polycystic ovaries) suffer from a disrupted follicular maturation, which is associated both with hormonal and ovulatory disruptions and with inadequately matured oocytes. The number of primary and secondary follicles is approximately twice as high here as in the normal ovary (Hughesden et al., Obstet. Gynecol. Survey 37, 1982, pp. 59-77).

There are indications that the early development stages of folliculogenesis (which relates to the development of primordial follicles to antral follicles) are gonadotropin-independent. It is not clearly explained how great the influence of known paracrine and autocrine factors is on early folliculogenesis (Elvin et al., Mol. Cell Endocrinol. 13, 1999, pp. 1035-1048; McNatty et al., J. Reprod. Fertil. Suppl. 54, 1999, pp. 3-16).

Gonadotropins such as FSH are mainly involved in the last development stages of folliculogenesis in follicular maturation, i.e., in the development of the early antral follicle to a mature follicle that can undergo ovulation.

The in-vivo and in-vitro infertility is preferably treated with gonadotropins (FSH and antiestrogens) (White et al., J. Clin. Endocrinol. Metab. 81, 1996, pp. 3821-3824). In in-vitro fertilization treatment, oocytes are removed from preovulatory antral follicles to be able to mature in vitro into an oocyte that can be fertilized. After fertilization and early embryonic development, one to three embryos are implanted in the uterus of the woman.

In many respects, the treatment with exogenic gonadotropins is accompanied by numerous risks and side effects. The greatest risk consists in an overstimulation of the ovaries, which in severe cases can represent a serious danger to life (OHSS = Ovarian Hyperstimulation Syndrome). Other side effects are the high costs of the in-vitro fertility treatment that must be paid by the couples. Negative side effects such as weight gain, bloatedness, nausea, vomiting and an as yet unknown long-term risk of developing cancer are attributed to the gonadotropin treatment.

One method to avoid the above-mentioned drawbacks and risks is to ensure the maturation and stimulation in vivo of follicular growth in the case of ovarian-induced infertility with a suitable active ingredient before treatment with exogenic gonadotropins begins.

### Estrogen Receptor Beta (ERβ)

Several years ago, estrogen receptor β (ERβ) was discovered as a second subtype of the estrogen receptor (Kuiper et al. (1996), Proc. Natl. Acad. Sci. 93: 5925-5930; Mosselman, Dijkema (1996) Febs Letters 392: 49-53; Tremblay et al. (1997), Molecular Endocrinology 11: 353-365). The expression pattern of ERβ differs from that of the ERα (Kuiper et al. (1996), Endocrinology 138: 863-870). ERβ thus predominates over ERα in the rat prostate, while ERα predominates over ERβ in the rat uterus. The highest concentrations of ERβ and mRNA were found in the ovaries (Couse et al. Endocrinology 138, 1997, pp. 4612-4613).

Other organ systems with comparatively higher ERβ-expression comprise the bones (Onoe, Y. et al., 1997, Endocrinology 138: 4509-4512), the vascular system (Register, T. C., Adams, M. R. 1998, J. Steroid Molec Biol 64: 187-191), the urogenital tract (Kuiper, G. J. M. et al. 1997, Endocrinology 138: 863-870), the gastrointestinal tract (Campbell-Thopson 1997, BBRC 240: 478-483), as well as the testis (Mosselmann, S. et al. 1996 FEBS Lett. 392, 49-53) including the spermatides (Shugrue et al. 1998, Steroids 63: 498-504). The tissue distribution suggests that estrogens regulation of organ functions via ERβ is highly relevant. The fact that ERβ is functional in this respect also follows by studies in ERα- (ERKO) or ERβ-(βERKO)-knockout mice: ovariectomy produces bone mass loss in ERKO-mice, which can be eliminated by estrogen substitution (Kimbro et al. 1998, Abstract OR7-4, Endocrine Society Meeting, New Orleans). Estradiol in the blood vessels of female ERKO mice also inhibits vascular media and smooth muscle cell proliferation (Iafrati, M. D. et al. 1997, Nature Medicine 3: 545-548). These protective actions of estradiol are carried out in the ERKO mouse presumably via ERβ.

The fact that ERα and ERβ have a functionally different action was confirmed after successful production of ERKO and βERKO mice. ERα consequently plays an important role in the adult uterus, in mammary gland tissue, in the negative regulation of the gonadotropin activity, while ERα is mainly bonded in the processes of ovarian physiology, especially that of folliculogenesis and ovulation (Couse et al., Endocrine Reviews 20, 1999, pp. 358-417).

Observations of βERKO mice provide an indication on a function of ERβ in the prostate and bladder: in the case of older male mice, symptoms of prostate and bladder hyperplasia occur (Krege, J. H. et al. 1998, Proc Natl Acad Sci 95: 15677-15682). In addition, female ERKO mice (Lubahn, D. B. et al. 1993, Proc Natl Acad Sci 90: 11162-11166) and male ERKO mice (Hess, R. A. et al. 1997, Nature 390: 509-512) as well as female βERKO mice (Krege, J. H., 1998, Proc Natl Acad Sci 95: 15677-15682) have fertility disorders. Consequently, the important function of estrogens with respect to maintaining testis and ovary functions as well as fertility is confirmed.

It is possible to achieve a selective estrogenic action on specific target organs by subtype-specific ligands based on the different tissue or organ distribution of the two subtypes of the ERs. Substances with a preference for ERβ compared to ERα in the in-vitro receptor binding test were described by Kuiper et al. (Kuiper et al. (1996), Endocrinology 138: 863-870). A selective action of subtype-specific ligands of the estrogen receptor on estrogen-sensitive parameters in vivo was not previously shown.

The patent application WO 01/77139 Al describes 8β-substituted estratrienes wherein R⁸ means a straight-chain or branched-chain, optionally partially or completely halogenated alkyl or alkenyl radical with up to 5 carbon atoms, an ethinyl- or prop-1-inyl radical, as pharmaceutical active ingredients that have in vitro a higher affinity to estrogen receptor preparations of rat prostates than to estrogen receptor preparations of rat uteri, their production, their therapeutic use and pharmaceutical dispensing forms that contain the said compounds. The compound 3-methoxy-8-vinyl-estra-1,3,5(10)-trien-17-one (Example 6) is also described in WO01/77139.

Compounds with potent estrogen activity and a higher affinity to estrogen receptor preparations of rat prostates than to estrogen receptor preparations of rat uteri for the production of medicaments are highly needed in the present technical field.

### Object of the Invention

The object of this invention is therefore to prepare compounds that have in vitro a high dissociation with respect to the binding to estrogen receptor preparations from rat prostates and rat uteri. The compounds are to show in vitro a higher affinity to estrogen receptor preparations from rat prostates than to estrogen receptor preparations from rat uteri.
The compounds according to the present invention have a high potent estrogen activity and efficacy, namely
a higher binding affinity to rat prostate estrogen receptor and a better dissociation regarding binding to rat prostate versus rat uterus estrogen receptor with respect to the known compounds. The compounds according to the invention are to produce enhanced fertility in the ovary while at the same time affecting the uterus very little in cases of ovarian-associated infertility.

The advantageous profile of the compounds according to the invention was achieved by the specific combination of the substituents R⁸, R¹³, R¹⁶, R¹⁷ and R^{17'}.

According to the invention, the object above is achieved by the provision of 8β-substituted estra-1,3,5(10)-triene derivatives of general formula I 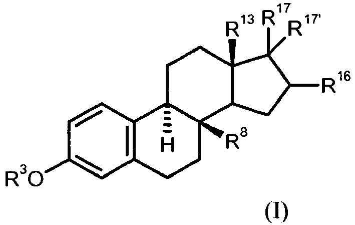 in which radicals R³, R⁸, R¹³, R¹⁶ as well as R¹⁷ and R^{17'}, independently of one another, have the following meaning:
R³ means a hydrogen atom or a group R¹⁸, in which
   R¹⁸ means a straight-chain or branched-chain, saturated or unsaturated C₁-C₆-alkyl radical, a trifluoromethyl group,
      an aryl, heteroaryl or aralkyl radical optionally substituted with at least one radical independently chosen from a methyl, ethyl, trifluoromethyl, pentafluoroethyl, trifluoromethylthio, methoxy, ethoxy, nitro, cyano, halo, hydroxy, amino, mono(C₁-C₈-alkyl) or di(C₁-C₈-alkyl)amino whereby both alkyl groups are identical or different, di(aralkyl)amino whereby both aralkyl groups are identical or different, carboxyl, carboxyalkoxy, C₁-C₂₀-acyl or C₁-C₂₀-acyloxy as substituents;
      an acyl radical -C(=O)R¹⁹, in which R¹⁹ is a straight-chain or branched-chain of a C₁-C₁₀ -alkyl radical that is saturated or unsaturated in up to three places and is partially or completely halogenated, or
   R¹⁸ means a group R²⁰SO_{2'} in which
      R is an R²¹R²²N group, whereby R²¹ and R²², independently of one another, mean a hydrogen atom, a C₁-C₅ -alkyl radical, a group -C(=O)R²³, in which R means a unsubstituted or substituted, straight-chain or branched-chain C₁-C₁₀ -alkyl radical that is saturated or unsaturated in up to three places and is partially or completely halogenated, a cyclopropyl, cyclobutyl, cyclopentyl, cyclohexyl or cycloheptyl group, a C₄-C₁₅-cycloalkylalkyl radical with 3 to 7 carbon atoms in the cycloalkyl portion and with an alkyl portion of up to 8 carbon atoms or an aryl, heteroaryl or aralkyl radical, optionally substituted with at least one radical independently chosen from a methyl, ethyl, trifluoromethyl, pentafluroethyl, trifluoromethylthio, methoxy, ethoxy, nitro, cyano, halo- , hydroxy, amino, mono(C₁-C₈-alkyl) or di(C₁-C₈-alkyl) amino, whereby both alkyl groups are identical or different, di(aralkyl)amino, whereby both aralkyl groups are identical or different, carboxyl, carboxyalkoxy, C₁ -C₂₀-acyl or C₁ -C₂₀-acyloxy groups as substituents; or, together with the N atom, a polymethylenimino radical with 4 to 6 carbon atoms or a morpholino radical,
      R⁸ is a straight-chain or branched-chain alkenyl or alkinyl radical with 2 to 6 carbon atoms, which optionally can be partially or completely fluorinated,
      R¹³ is a methyl group or an ethyl group,
      R¹⁶ is a fluorine atom in the ∝ position,
      R¹⁷, and R^{17'}, in each case independently of one another, are a hydrogen atom and a hydroxy group; or
         a hydrogen atom and a group R¹⁸O-, R²⁰SO₂- or OC(O)R²³ with R¹⁸, R²⁰ and R²³ in each case in the meaning that is indicated under R³.

A particular embodiment of the present invention are the compounds of general formula I, in which R³ is a hydrogen atom.

According to a further embodiment of the invention, compounds of general formula I comprise R⁸ being a vinyl, ethinyl or prop-1-inyl group.

Other possible forms of embodiment of the present invention are defined by the depending claims.

Compounds of general formula I in which R¹⁷ and R^{17'} are a hydrogen atom and a hydroxy group atom are further preferred.

Compounds of general formula I in which R¹⁶ is in α-position or compounds of general formula I in which R¹⁶ is in β-position are equally preferred forms of embodiment of the invention.

Furthermore, a particular embodiments of the present invention are the compounds of general formula I in which R⁸ is a vinyl, ethinyl or prop-1-inyl group, R¹⁶ is a fluorine atom, R¹⁷ and R^{17'} are independently from one another a hydrogen atom and a hydroxy group atom.

Compounds of general formula I in which R¹⁷ and/or R^{17'} are a hydrogen atom and a group R¹⁸-O- or R¹⁹SO₂-O- with R¹⁸ and R¹⁹ in each case in the meaning that is indicated under R³ are further particular form of embodiment of the present invention.

Another variant of the invention in particular calls for compounds in which R stands for a group R¹⁸O- or R²⁰SO₂-O- with R¹⁸ and R²⁰ in each case in the meaning that is indicated under R³.

Compounds according to the present invention are:
8β-vinyl-16α-fluoro-estra-1,3,5(10)-triene-3,17α-diol
8β-vinyl-16α-fluoro-estra-1,3,5(10)-triene-3,17β-diol

As halo- or halogen, a fluorine, chlorine, bromine or iodine atom is intended according to the present invention

According to the above definition R¹⁸ is, for example, a methyl, ethyl, propyl, isopropyl, butyl, isobutyl, tert.-butyl, pentyl, isopentyl, neopentyl, or hexyl radical.

Alkoxy groups OR¹⁸ in the compounds of general formula I in each case can contain an alkyl radical according to the definition given above, whereby methoxy, ethoxy, propoxy, isopropoxy and t-butyloxy groups are preferred alkoxy radical.

Representatives of the C₁-C₅-alkyl radicals R²¹ and R²² are methyl, ethyl, propyl, isopropyl, butyl, isobutyl, tert-butyl, pentyl, isopentyl and neopentyl.

As representatives of straight-chain or branched-chain C₁-C₁₀ -alkyl radicals R²³, for example, methyl, ethyl, propyl, isopropyl, butyl, isobutyl, tert-butyl, pentyl, isopentyl, neopentyl, heptyl, hexyl, and decyl can be mentioned; methyl, ethyl, propyl and isopropyl are preferred.

As a C₃-C₇-cycloalkyl group, a cyclopropyl, cyclobutyl, cyclopentyl, cyclohexyl or cycloheptyl group can be mentioned.

A C₄-C₁₅-cycloalkylalkyl radical has 3 to 7 carbon atoms in the cycloalkyl portion; typical representatives are the cycloalkyl groups that are mentioned directly above. The alkyl portion has up to 8 carbon atoms.

As examples of a C₄-C₁₅-cycloalkylalkyl radical, the cyclopropylmethyl, cyclopropylethyl, cyclopentylmethyl, cyclopentylpropyl groups, etc., can be mentioned.

In terms of this invention, an aryl radical is a phenyl, 1- or 2-naphthyl radical; the phenyl radical is preferred.

Examples of a heteroaryl radical according to the present invention are the 2-, 3- or 4-pyridinyl, the 2- or 3-furyl, the 2- or 3-thienyl, the 2-or 3-pyrrolyl, the 2-, 4- or 5-imidazolyl, the pyrazinyl, the 2-, 4- or 5-pyrimidinyl or 3- or 4-pyridazinyl radical.

As substituents that can be present on an aryl or heteroaryl radical, are a methyl-, ethyl-, trifluoromethyl-, pentafluoroethyl-, trifluoromethylthio-, methoxy-, ethoxy-, nitro-, cyano-, halogen- (fluorine, chlorine, bromine, iodine), hydroxy-, amino-, mono(C₁-C₈-alkyl) or di(C₁-C₈-alkyl)amino, whereby both alkyl groups are identical or different, di(aralkyl)amino, whereby both aralkyl groups are identical or different, carboxyl, carboxyalkoxy, C₁-C₂₀-acyl or C₁-C₂₀-acyloxy groups can be mentioned.

An aralkyl radical is a radical that contains in the ring up to 14, preferably 6 to 10 C atoms, and in the alkyl chain 1 to 8, preferably 1 to 4, C atoms. Thus, as aralkyl radicals, for example, benzyl, phenylethyl, naphthylmethyl, naphthylethyl, furylmethyl, thienylethyl, and pyridylpropyl are suitable.

The alkyl groups and radicals can be partially or completely substituted by 1-5 halogen atoms, hydroxy groups or C₁-C₄-alkoxy groups.

A C₂-C₆-alkenyl radical is preferably a vinyl or allyl radical

A C₂-C₆-alkinyl radical is preferably defined as an ethinyl radical or a prop-1-inyl radical.C₁₋₁₀-Acyl radicals mean, for example, acetyl, propionyl, butyryl, valeroyl, isovaleroyl, pivaloyl, hexanoyl, octyl, nonyl, or decanoyl.

Suitable as such carboxylic acids for esterification resulting in esters falling within the scope of claim 1 are, for example:
- Monocarboxylic acids: formic acid, acetic acid, propionic acid, butyric acid, isobutyric acid, valeric acid, isovaleric acid, pivalic acid, acrylic acid, propionic acid, methacrylic acid, crotonic acid, isocrotonic acid Esterification with acetic acid, valeric acid or pivalic acid is preferred.

As prodrugs, the esters of the 8β-substituted estratrienes according to the invention and falling within the scope of claim 1 have advantages compared to the unesterified active ingredients with respect to their method of administration, their type of action, strength and duration of action.

Especially the sulfamates of 8β-substituted estratrienes according to the invention have pharmacokinetic and pharmacodynamic advantages. Related effects were already described in other steroid-sulfamates (J. Steroid Biochem. Molec. Biol, 55, 395-403 (1995); Exp. Opinion Invest. Drugs 7, 575-589 (1998)).

In this patent application, steroids based on a 8β-substituted estra-1,3,5(10)-triene skeleton are described for the treatment of estrogen receptor β-mediated disorders and conditions as selective estrogens, which have in-vitro dissociation with respect to their binding to estrogen receptor preparations from rat prostates and rat uteri and which have in vivo preferably a dissociation with respect to ovary action in comparison to uterus action.

The ERβ-specific compounds according to the present invention are to mediate in vivo a profertility action in the ovary. At the same time, the compounds are to exhibit a dissociation with respect to ovary action in comparison to uterus action.

It was found that the 8β-substituted estra-1,3,5(10)-trienes according to general formula I are suitable as selective estrogens for the treatment of various conditions and disorders that are characterized by a higher content of estrogen receptor β than estrogen receptor α in the corresponding target tissue or target organ. Said compounds have improved potency and metabolic stability.

The invention also relates to pharmaceutical preparations that contain at least one compound of general formula I (or physiologically compatible addition salts with organic or inorganic acids thereof) and the use of the compounds of general formula I for the production of pharmaceutical agents, especially for the indications mentioned below.

The new selective estrogens that are described here can be used as individual components in pharmaceutical preparations or in combination especially with gestagens. Part of the present invention is the combination of selective estrogens with ERα-selective antiestrogens that are peripherally-selectively active, i.e., that do not pass through the blood-brain barriers, as well as with selective estrogen receptor modulators (SERM).

The ERβ-selective compounds according to the invention can be used in particular for the production of pharmaceutical agents for treating fertility disorders, for prevention and therapy of prostate hyperplasia, for prevention and treatment of hormone-deficiency-induced mood swings in women and men and for use in hormone replacement therapy (HRT) in men and women.

Furthermore, the compounds according to the invention have antiproliferative effects in models of colon and intestinal hyperplasia and can therefore be administered for the prevention and treatment of diseases associated with colon and intestinal epithelium proliferation like for the treatment and the prevention of cancer.

The ERβ-specific compounds according to the present invention can be advantageously used for the selective stimulation of hair growth.

A therapeutic product that contains an estrogen and a pure antiestrogen for simultaneous, sequential or separate use for the selective estrogen therapy of perimenopausal or postmenopausal conditions is already described in EP-A 0 346 014.

Because of their dissociation of action in the ovary in comparison to the action of the uterus, the substances and the pharmaceutical agents that contain them are especially suitable for the treatment in the case of ovarian dysfunction that is caused by surgery, medication, etc., such as female infertility for stimulation of folliculogenesis for treatment by itself in terms of enhanced fertility, for supporting in-vitro fertility treatment (IVF) in connection with an in-vivo treatment and for treatment of ovarian-induced disorders in later age ("late fertility") as well as for treatment of hormone-deficiency-induced symptoms.

The compounds of this invention are also suitable for therapy of ovarian diseases such as polycystic ovarian syndrome, POF (premature ovarian failure) syndrome, and ovulation disorders.

Finally, the compounds of general formula I can be used in connection with selective estrogen receptor modulators (SERM) or raloxifene, specifically in particular for use in hormone replacement therapy (HRT) and for treatment of gynecological disorders.

The 8β-substituted estratrienes according to the invention are also suitable as individual components for the treatment of perimenopausal and postmenopausal symptoms, in particular hot flashes, sleep disturbances, irritability, mood swings, incontinence, vaginal atrophy and hormone-deficiency-induced mental disorders. The above 8β-substituted estratrienes are also suitable for hormone substitution and for the therapy of hormone-deficiency-induced symptoms in ovarian dysfunction that is caused by surgery, medication, etc.

In addition, the 8β-substituted estratrienes according to the invention can also be used to prevent cardiovascular diseases, in particular vascular diseases such as arteriosclerosis, high blood pressure, hypertensive heart disease and to prevent hormone-deficiency-induced neurodegenerative diseases, such as Alzheimer's disease, as well as hormone-deficiency-induced impairment of memory and learning capacity.

In addition, the compounds according to the present invention can be used as active ingredients in preparations for treating inflammatory diseases and diseases of the immune system, in particular autoimmune diseases, such as, e.g., rheumatoid arthritis, multiple sclerosis, lupus, Crohn's disease and other inflammatory intestinal diseases, inflammatory diseases of the skin, such as psoriasis, as well as for treating endometriosis. Building on the evidence from preclinical models of human inflammatory diseases, the ERβ-specific compounds can therfore be used for the prevention and treatment of the diseases mentioned above (Heather, H.A.; Mol Endocrinol. 2007 Jan;21(1):1-13. Epub 2006 Mar 23. Review)..

In addition, the compounds are effective against inflammatory diseases of the respiratory system, the lungs and bronchial tubes, such as, e.g., asthma.

The medication is suitable for therapy and prophylaxis of estrogen-deficiency-induced diseases both in women and in men.
The present compounds are also suitable for prevention and therapy of prostate hyperplasia.

The compounds can be further used for prophylaxis and therapy of humans age-related dysfunctions or diseases. In particular, they can be used for prevention and treatment of an age-related drop of androgens, such as testosterone and DHEA, as well as of the growth hormone.

The amount of a compound of general formula I that is to be administered fluctuates within a wide range and can cover any effective amount. On the basis of the condition that is to be treated and the type of administration, the amount of the compound that is administered can be 0.01 µg/kg - 100 mg/kg of body weight, preferably 0.04 µg/kg - 1 mg/kg of body weight, per day.

In humans, this corresponds to a dose of 0.8 µg to 8 g, preferably 3.2 µg to 80 mg, daily.

According to the invention, a dosage unit contains 1.6 µg to 2000 mg of one or more compounds of general formula I.

The compounds according to the invention and the acid addition salts are suitable for the production of pharmaceutical compositions and preparations. The pharmaceutical compositions or pharmaceutical agents contain as active ingredients one or more of the compounds according to the invention or their acid addition salts, optionally mixed with other pharmacologically or pharmaceutically active substances. The production of the pharmaceutical agents is carried out in a known way, whereby the known and commonly used pharmaceutical adjuvants as well as other commonly used vehicles and diluents can be used.

As such vehicles and adjuvants, for example, those are suitable that are recommended or indicated in the following bibliographic references as adjuvants for pharmaceutics, cosmetics and related fields: Ullmans Encyklopädie der technischen Chemie [Ullman's Encyclopedia of Technical Chemistry], Volume 4 (1953), pages 1 to 39; Journal of Pharmaceutical Sciences, Volume 52 (1963), page 918 ff., issued by Czetsch-Lindenwald, Hilfsstoffe für Pharmazie und angrenzende Gebiete [Adjuvants for Pharmaceutics and Related Fields]; Pharm. Ind., Issue 2, 1961, p. 72 and ff.: Dr. H. P. Fiedler, Lexikon der Hilfsstoffe für Pharmazie, Kosmetik und angrenzende Gebiete [Dictionary of Adjuvants for Pharmaceutics, Cosmetics and Related Fields], Cantor KG, Aulendorf in Württemberg 1971.

The compounds can be administered orally or parenterally, for example intraperitoneally, intramuscularly, subcutaneously or percutaneously. The compounds can also be implanted in the tissue.

For oral administration, capsules, pills, tablets, coated tablets, etc., are suitable. In addition to the active ingredient, the dosage units can contain a pharmaceutically compatible vehicle, such as, for example, starch, sugar, sorbitol, gelatin, lubricant, silicic acid, talc, etc.

For parenteral administration, the active ingredients can be dissolved or suspended in a physiologically compatible diluent. As diluents, very often oils with or without the addition of a solubilizer, a surfactant, a suspending agent or an emulsifying agent are used. Examples of oils that are used are olive oil, peanut oil, cottonseed oil, soybean oil, castor oil and sesame oil.

The compounds can also be used in the form of a depot injection or an implant preparation, which can be formulated so that a delayed release of active ingredient is made possible.

As inert materials, implants can contain, for example, biodegradable polymers, or synthetic silicones such as, for example, silicone rubber. In addition, for percutaneous administration, the active ingredients can be added to, for example, a patch.

For the production of intravaginal systems (e.g., vaginal rings) or intrauterine systems (e.g., pessaries, coils, IUDs, Mirena^{(R)}) that are loaded with active compounds of general formula I for local administration, various polymers are suitable, such as, for example, silicone polymers, ethylene vinyl acetate, polyethylene or polypropylene.

To achieve better bio-availability of the active ingredient, the compounds can also be formulated as cyclodextrin clathrates. For this purpose, the compounds are reacted with α-, β-, or γ-cyclodextrin or derivatives of the latter (PCT/EP95/02656).

According to the invention, the compounds of general formula I can also be encapsulated with liposomes.

### Methods

### Estrogen Receptor Binding Studies:

The binding affinity of the new selective estrogens was tested in competitive experiments with use of ³H-estradiol as a ligand to estrogen receptor preparations from rat prostates and rat uteri. The preparation of prostate cytosol and the estrogen receptor test with prostate cytosol was carried out as described by Testas et al. (1981) (Testas, J. et al., 1981, Endocrinology 109: 1287-1289).

The preparation of rat uterus cytosol as well as the receptor test with the ER-containing cytosol were basically performed as described by Stack and Gorski, (1985) (Stack, Gorski 1985, Endocrinology 117, 2024-2032) with some modifications as described in Fuhrmann et al. (1995) (Fuhrmann, U. ct al. 1995, Contraception 51: 45-52).

The substances that are described here have higher binding affinity to the estrogen receptor of rat prostates than to estrogen receptors of rat uteri. In this case, it is assumed that ERβ predominates in the rat prostates over ERα, and ERα predominates in rat uteri over ERβ. Table 1 shows that the ratio of the binding to prostate and uterus receptors qualitatively coincides with the quotient of relative binding affinity (RBA) to human ERβ and ERα of rats (according to Kuiper et al. (1996), Endocrinology 138: 863-870) (Table 1).

**Table 1**

| **Estrogen** | **Structure** | **hERα RBA*** | **hERβ RBA*** | **ERβ/ ERα** | **Rat uterus ER(RBA)** | **Rat prost. ER(RBA)** | **prost. ER/uterus ER** |
|---|---|---|---|---|---|---|---|
| **Estradiol** | 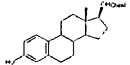 | 100 | 100 | 1 | 100 | 100 | 1 |
| **Estrone** | 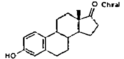 | 60 | 37 | 0.6 | 3 | 2 | 0.8 |
| **17α-Estra-diol** | 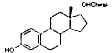 | 58 | 11 | 0.2 | 2.4 | 1.3 | 0.5 |
| **Estriol** | 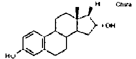 | 14 | 21 | 1.5 | 4 | 20 | 5 |
| **5-Androstene-diol** | 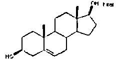 | 6 | 17 | 3 | 0.1 | 5 | 50 |
| **Genisteine** | 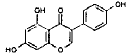 | 5 | 36 | 7 | 0.1 | 10 | 100 |
| **Coumestrol** | 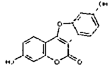 | 94 | 185 | 2 | 1.3 | 24 | 18 |

| | | | | | | | |
|---|---|---|---|---|---|---|---|
| *Cited from: Kuiper et al. (1996), Endocrinology 138: 863-870 | | | | | | | |

Table 2 shows the results for one of the 8β-vinyl-estra-1,3,5(10)-triene-3,17-diol derivatives (compounds **1**) according to the invention. Compound **2** (8β-vinyl-estra-1,3,5(10)-triene-3,17-diol is shown as a reference.

**Table 2**

| **Compound** | Rat uterus ER(RBA) | Rat prost. ER(RBA) | prost. ER/uterus ER |
|---|---|---|---|
| 8β-Vinyl-estra-1,3,5(10)-16α-fluoro-3,17β-diol (**1**) | 1.7 | 167 | 98 |
| 8β-Vinyl-estra-1,3,5(10)-3,17β-diol (**2**) | 0.7 | 63 | 90 |

Compounds **1** according to the invention as well as compound 2 show a higher binding affinity to the estrogen receptor of rat prostates than to the estrogen receptor of rat uteri. Compound 1 is superior to compound 2 by a higher binding affinity to rat prostate estrogen receptor and by a better dissociation regarding binding to rat prostate versus rat uterus estrogen receptor.

In addition, the predictability of the prostate-ER versus the uterus-ER test system is confirmed with respect to tissue-selective action by in-vivo studies. Substances with a preference for prostate-ER are dissociated in vivo preferably with respect to ovary and uterus action as well as pituitary gland action in favor of action on the ovary.

### Studies for Dissociation of Action of the Uterus and Pituitary Gland

The studies with respect to the action on uterus growth and ovulation (indirect effect by influencing the secretion of pituitary gland hormones) are performed on adult female rats (body weight of 220-250 g). The substances are subcutaneously administered four times on four consecutive days. The first administration is carried out in the metestrus. One day after the last administration, the autopsy is carried out. The number of oocytes in the Fallopian tube (effect on the ovulation) as well as the uterus weight are determined.

While estradiol produces a dose-dependent ovulation inhibition and an increase in uterus weight with an ED₅₀ of 0.004 mg/kg of body weight, the substance according to the invention does not exert any effect on pituitary gland and uterus weight.

### Ovary Studies:

The compounds are tested in vivo on hypophysectomized juvenile rats. In a modification of this operative method, a GnRH antagonist (Cetrorelix) is administered to the animals. It is examined whether the substance stimulates follicular proliferation (maturation) in the ovary. The ovary weight is the measurement parameter.

In each case, five animals (body weight 40-50 g) are assigned randomly to the treatment groups. The animals are fed at libitum with a standard diet (altromin) in Makrolon cages in airconditioned rooms with a lighting program (12 hours of darkness, 12 hours of light) and are given acidified tap water to drink. For the s.c administration, the test substance as well as the control substance (estradiol E2) are dissolved in benzylbenzoate/castor oil (1 +4 v/v).

Juvenile female rats are either hypophysectomized on day 0 and subcutaneously treated (administration 1 x daily) from day 1 to day 4 with estradiol, the compound according to the invention, or subcutaneously treated (administration 1 x daily) with a vehicle (castor oil/benzyl benzoate). In the modified version of the method, 0.5 mg/animal/day of Cetrorelix is administered to the animals simultaneously with the compound according to the invention or the vehicle and the control substance estradiol over four days of treatment. In both cases, the animals are sacrificed 24 hours after the last administration, and ovary weight as well as follicle stages are determined.

The compounds according to the invention thus shows a clear dissociation of action in the ovary in comparison to the uterus action and the pituitary gland action and is excellently suited for the treatment of female infertility, because of its follicle-stimulating action.

### Production of the Compounds According to the Invention

The compounds of general formula I according to the invention are produced as described in the examples. Additional compounds of general formula I can be obtained by an analogous procedure using reagents that are homologous to the reagents that are described in the examples.

Etherification and/or esterification of free hydroxy groups is carried out according to methods that are common to one skilled in the art.

The compounds according to the invention can be present in carbon atoms 16 and 17 as α,β-stereoisomers. In the production of compounds according to the described processes, the compounds in most cases accumulate as mixtures of the corresponding α,β-isomers. The mixtures can be separated by, for example, chromatographic processes.

According to general formula I, possible substituents can already be present in final form or in the form of a precursor even in the starting product, a substituted estrone already corresponding to the desired end product.

17-Substituents are also introduced according to known processes by nucleophilic addition of the desired substituent or a reactive precursor thereof and are optionally further built up.

The 8β-substituted estratriene-carboxylic acid esters according to the invention are produced from the corresponding hydroxy steroids analogously to processes that are also known (see, e.g., Pharmazeutische Wirkstoffe, Synthesen, Patente, Anwendungen [Pharmaceutical Active Ingredients, Syntheses, Patents, Applications]; A. Kleemann, J. Engel', Georg Thieme Verlag Stuttgart 1978, Arzneimittel, Fortschritte [Pharmaceutical Agents, Improvements] 1972 to 1985; A. Kleemann, E. Lindner, J. Engel (Editors), VCH 1987, pp. 773-814).

The estratriene-sulfamates according to the invention are available in a way that is known in the art from the corresponding hydroxy steroids by esterification with sulfamoyl chlorides in the presence of a base (Z. Chem. 15, 270-272 (1975); Steroids 61, 710-717 (1996)).

Subsequent acylation of the sulfamide group results in the (N-acyl)sulfamates according to the invention, for which pharmacokinetic advantages were already detected in the case of the absence of an 8-substituent (cf. DE 195 40 233 A1).

The regioselective esterification of polyhydroxylated steroids with N-substituted and N-unsubstituted sulfamoyl chlorides is carried out according to partial protection of those hydroxyl groups that are to remain unesterified. Silyl ethers have turned out to be protective groups with selective reactivity that is suitable for this purpose, since these silyl ethers are stable under the conditions of sulfamate formation, and the sulfamate group remains intact when the silyl ethers are cleaved again for regeneration of the residual hydroxyl group(s) still contained in the molecule (Steroids 61, 710-717 (1996)). The production of the sulfamates according to the invention with one or more additional hydroxyl groups in the molecule is also possible in that the starting material is suitable hydroxy-steroid ketones. First, depending on the goal, one or more hydroxyl groups that are present are subjected to sulfamoylation. Then, the sulfamate groups optionally can be converted with a desired acyl chloride in the presence of a base into the (N-acyl)sulfamates in question. The now present oxosulfamates or oxo-(N-acyl)sulfamates are converted by reduction into the corresponding hydroxysulfamates or hydroxy-(N-acyl)sulfamates (Steroids 61, 710-717 (1996)). Sodium borohydride and the borane-dimethyl sulfide complex are suitable as suitable reducing agents.

The introduction of variable substituents in rings D of the estratriene skeleton, particularly the halogen atom (for example a fluorine atom) at C-atom 16, can basically be carried out according to the chemical teaching that is known to one skilled in the art, with which the corresponding estratriene derivatives that are not substituted in 8-position are produced (see, i.a.: Steroide [Steroids], L. F. Fieser, M. Fieser, Verlag Chemie, Weinheim/Bergstr., 1961; Organic Reactions in Steroid Chemistry, J. Fried, J. A. Edwards, Van Nostrand Reinhold Company, New York, Cincinnati, Toronto, London, Melbourne, 1972; Medicinal Chemistry of Steroids, F. J. Zeelen, Elsevier, Amsterdam, Oxford, New York, Tokyo, 1990).

Substituents according to general formula I can also be introduced in the stage of estratrienes that are already substituted in 8-position, according to known methods in the art. This can be useful or necessary especially in the case of multiple substitutions of the desired final compound.

Characteristic, but not limiting synthesis processes, which are useful for providing representative substitution patterns on the estrone skeleton, also in combination with several substituents, are found in, for example: C(1) J. Chem. Soc. (C) 1968, 2915; C(7) Steroids 54, 1989, 71; C(8α) Tetrahedron Letters 1991, 743; C(8β) Tetrahedron Letters 1964, 1763; J. Org. Chem. 1970, 35, 468; C(11) J. Steroid Biochem. 31, 1988, 549; Tetrahedron 33, 1977, 609 and J. Org. Chem. 60, 1995, 5316; C(9) DE-OS 2035879; J. Chem. Soc. Perk. 1 1973, 2095; C(15) J. Chem. Soc. Perk. 1 1996, 1269.); C(13α) Mendeleev Commun. 1994, 187; C(14β) Z. Chem. 23, 1983,410.

The examples below are used for a more detailed explanation of the invention.

Analogously to the degradation of the 8β-vinyl grouping, other compounds of general formula I can be obtained with use of reagents that are homologous to the reagents that are described in the examples.

Etherification and/or esterification of free hydroxy groups is carried out according to the methods that are common to one skilled in the art.

### Example 1

### 8β-Vinyl-16α-Fluoro-estra-1,3,5(10)-triene-3,17-diol

### 3-Methoxy-16α-fluor-8-vinyl-estra-1,3,5(10)-trien-17-one

7,3 g (23,5 mmol) of 3-methoxy-8-vinyl-estra-1,3,5(10)-trien-17-one in 65 ml THF are added dropwise under argon to 30 ml of a 2M solution of lithium-diisopropylamide (60,0 mmol) in THF / Heptan / Ethylbenzol, cooled down at -78°C, and then 16 ml (115,4 mmol) Triethylamin and 7,6 ml (59,9 mmol) chlortrimethylsilane are added one after the other. The reaction mixture is subsequently warmed up to room temperature in 1,5 h, washed with sodium bicarbonate solution, and extracted with n-Hexane. The collected organic phases are washed with water, drayed over sodium sulphate and concentrated by evaporation in a vacuum. The obtained (3-methoxy-8-vinyl-estra-1,3,5(10),16-trien-17-yloxy)-trimethylsilane crude product (12 g of a yellowish liquid residue) is used without any further purification in the next step.
*R_{f}=* 0,54 (Cyclohexane / Ethyl Acetate = 8/2)

6,8 g of the (3-Methoxy-8-vinyl-estra-1,3,5(10),16-tetraen-17-yloxy)-trimethylsilane crude product are dissolved in 50 ml methylenchloride, combined with 5g (15,9mmol) N-fluordibenzensulfonimide and mixed for 16 hours at room temperature in the absence of light. 25 ml 2N hydrochloric acid are added, the organic phase is separated and extracted several times with methylchloride. The collected organic phases are washed with a sodium bicarbonate solution, with water and with a saturated sodium chloride solution one after the other, drayed over magnesium sulphate and concentrated in vacuo. The obtained crude product (8.43 yellowish-brown oil) is purified by chromatography on silica gel (19/1 cyclohexane/ethyl acetate). In this way 1.65g (38%, colourless foam) of 3-methoxy-16α-fluor-8-vinyl-estra-1,3,5(10)-trien-17-one and 2,08g (51% colourless foam) of 3-methoxy-8-vinyl-estra-1,3,5(10)-trien-17-one are obtained.

3-Methoxy-16α-fluor-8-vinyl-estra-1,3,5(10)-trien-17-one 1H-NMR (CDCl3): δ = 0,96 (s, 3H; 18-CH3), 3,76 (s, 3H; OMe), 5,01 (d, 1H; CH=CH2), 5,05/5,18 (d, 1H; 16-H), 5,13 (d, 1H; CH=CH2), 5,54 (dd, 1H; CH=CH2), 6,59 (d, 1H; 4-H), 6,69 (dd, 1H; 1-H, 2-H), 7,15 (d, 1H; 1-H, 1-H).

### 3-Methoxy-16α-fluor-8-vinyl-estra-1,3,5(10)-trien-17α-ol und 3-methoxy-16α-fluor-8-vinylestra-1,3,5(10)-trien-17β-ol

1,65 g (5,0 mmol) of 3-Methoxy-16α-fluor-8-vinyl-estra-1,3,5(10)-trien-17-one are dissolved in a mixture of 150 ml of THF and 150 ml of Methanol, cooled at 5°C and combined to 1,9 g (50,2 mmol) of sodiumborohydride. During the warming up to room temperature in 1,5h, the reaction mixture is stirred. 10 ml of acetic acid are then added and the solution finally dried in vacuo. The residue is redissolved in water and extracted several times with ethyl acetate. The organic phases are collected, dried with magnesium sulfate and concentrated in vacuo.

The obtained crude product is separated by chromatography on silica gel (Cyclohexane / Methyltert.-Buthylether= 13/1). According to this procedure 0,67 g (40%) of 3-methoxy-16α-fluor-8-vinyl-estra-1,3,5(10)-trien-17α-ol and 1,00 g (60%) 3-of methoxy-16α-fluor-8-vinyl-estra-1,3,5(10)-trien-17β-ol are obtained in the form of a colorless foam.
3-Methoxy-16α-fluor-8-vinyl-estra-1,3,5(10)-trien-17α-ol 1H-NMR (CDCl3): δ=0,72 (s, 3H; 18-CH3), 3,72 (d, 1H, 17-H), 3,75 (s, 3H; OMe), 4,90 (d, 1H; CH=CH2), 5,04 (d, 1H; CH=CH2), 5,14 / 5,28 (dd, 1H; 16-H), 5,51 (dd, 1H; CH=CH2), 6,57 (d, 1H; 4-H), 6,67 (dd, 1H; 1-H, 2-H), 7,16 (d, 1H; 1-H, 1-H).

3-Methoxy-16α-fluor-8-vinyl-estra-1,3,5(10)-trien-17β-ol
1H-NMR (CDCl3): δ = 0,79 (s, 3H; 18-CH3), 3,75 (s, 3H; OMe), 3,81 (d, 1H, 17-H), 4,82 / 4,95 (dd, 1H; 16-H), 4,89 (d, 1H; CH=CH2), 5,06 (d, 1H; CH=CH2), 5,51 (dd, 1H; CH=CH2), 6,57 (d, 1H; 4-H), 6,68 (dd, 1H; 1-H, 2-H), 7,15 (d, 1H; 1-H, 1-H).

### 16α-Fluor-8-vinyl-estra-1,3,5(10)-trien-3,17β-diol

690 mg (1,87 mmol) of tetrabutylammoniumiodide are added to a solution of 70 mg (0,21 mmol) 3-methoxy-16α-fluor-8-vinyl-estra-1,3,5(10)-trien-17β-ol in 9 ml of methylenchloride under argon. The reaction mixture is cooled at -78°C and then combined with 1,9 ml (1,9 mmol) of a 1M solution ofborotrichloride in methylenchloride. The mixture is stirred for 0,5 h at -78°C, finally warmed up at room temperature and quenched with water. The organic phase is separated, the water phase is extracted several times with methylenchloride, and the organic phases are collected, washed with a saturated sodium chloride solution, dried with magnesium sulfate and concentrated in vacuo.

The obtained crude product is separated by chromatography on silica gel (Cyclohexane / Ethyl Acetate = 9 / 1). 26 mg (39%) of 16α-Fluor-8-vinyl-estra-1,3,5(10)-trien-3,17β-diol are obtained according to this procedure.

16α-Fluor-8-vinyl-estra-1,3,5(10)-trien-3,17β-diol 1H-NMR (DMSO[D6]): δ = 0,64 (s, 3H; 18-CH3), 3,49 / 3,57 (t, 1H, 17-H), 4,70 / 4,84 (dd, 1H; 16-H), 4,85 (d, 1H; CH=CH2), 4,98 (d, 1H; CH=CH2), 5,19 (d, 1H, 17-OH), 5,46 (dd, 1H; CH=CH2), 6,37 (d, 1H; 4-H), 6,48 (dd, 1H; 1-H, 2-H), 6,99 (d, 1H; 1-H, 1-H), 8,93 (s, 1H, 3-OH).

### Example 2

### 16α-Fluor-8-vinyl-estra-1,3,5(10)-trien-3,17α-diol

295 mg (0,80 mmol) of tetrabutylammoniumiodide are added to a solution of 30 mg (0,09 mmol) of 3-methoxy-16α-fluor-8-vinyl-estra-1,3,5(10)-trien-17α-ol in 4 ml methylenchloride under argon. The reaction mixture is cooled at -78°C and then combined with 0,8 ml (0,8 mmol) of a 1M solution of borotrichloride in methylenchloride. The mixture is stirred for 0,5 h at -78°C, finally warmed up at room temperature and quenched with water. The organic phase is separated, the water phase is extracted several times with methylenchloride, and the organic phases are collected, washed with a saturated Sodium Chloride solution, dried with magnesium sulfate and concentrated in vacuo.

The obtained crude product is separated by chromatography on silica gel (Cyclohexane / Ethyl Acetate = 7 / 3). According to this procedure 27 mg (94%) 16α-Fluor-8-vinyl-estra-1,3,5(10)-trien-3,17α-diol are obtained.

16α-Fluor-8-vinyl-estra-1,3,5(10)-trien-3,17α-diol 1H-NMR (DMSO[D6]):δ = 0,63 (s, 3H; 18-CH3), 3,51 (t, 1H, 17-H), 4,82 (d, 1H, 17-OH), 4,85 (d, 1H; CH=CH2), 4,97 (d, 1H; CH=CH2), 5,05 / 5,18 (dd, 1H; 16-H), 5,47 (dd, 1H; CH=CH2), 6,37 (d, 1H; 4-H), 6,48 (dd, 1H; 1-H, 2-H), 6,99 (d, 1H; 1-H, 1-H), 8,97 (s, 1H, 3-OH).

## Claims

1. 8β-substituted estra-1,3,5(10)-triene derivatives of general formula I 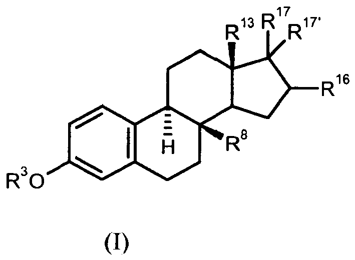 in which radicals R³, R⁸, R¹³, R¹⁶ as well as R¹⁷ and R^{17'}, independently of one another, have the following meaning:
R³ means a hydrogen atom or a group R¹⁸, in which
R¹⁸ means a straight-chain or branched-chain, saturated or unsaturated C₁-C₆-alkyl radical, a trifluoromethyl group,
an aryl, heteroaryl or aralkyl radical, optionally substituted with at least one radical independently chosen from a methyl, ethyl, trifluoromethyl, pentafluoroethyl, trifluoromethylthio, methoxy, ethoxy, nitro, cyano, halo, hydroxy, amino, mono(C₁-C₈-alkyl) or di(C₁-C₈-alkyl)amino whereby both alkyl groups are identical or different, di(aralkyl)amino whereby both aralkyl groups are identical or different, carboxyl, carboxyalkoxy, C₁-C₂₀-acyl or C₁-C₂₀-acyloxy as substituents;
an acyl radical -C(=O)R¹⁹, in which R¹⁹ is a straight-chain or branched-chain of a C₁-C₁₀ -alkyl radical atoms that is saturated or unsaturated in up to three places and is partially or completely halogenated, or
R¹⁸ means a group R²⁰SO₂, in which
R²⁰ is an R²¹R²² N group, whereby R²¹ and R²², independently of one another, mean a hydrogen atom, a C₁-C₅-alkyl radical, a group 23 23 -C(=O)R²³, in which R means a unsubstituted or substituted, straight-chain or branched-chain C₁-C₁₀ -alkyl radical that is saturated or unsaturated in up to three places and is partially or completely halogenated, a cyclopropyl, cyclobutyl, cyclopentyl, cyclohexyl or cycloheptyl group, a C₄-C₁₅-cycloalkylalkyl radical with 3 to 7 carbon atoms in the cycloalkyl portion and with an alkyl portion of up to 8 carbon atoms or an aryl, heteroaryl or aralkyl radical optionally substituted, with at least one radical independently chosen from a methyl, ethyl, trifluoromethyl, pentafluroethyl, trifluoromethylthio, methoxy, ethoxy, nitro, cyano, halo-, hydroxy, amino, mono(C₁-C₈-alkyl) or di(C₁-C₈-alkyl) amino whereby both alkyl groups are identical or different, di(aralkyl)amino whereby both aralkyl groups are identical or different, carboxyl, carboxyalkoxy, C₁ -C₂₀-acyl or C₁ -C₂₀-acyloxy groups as substituents; or,
together with the N atom, a polymethylenimino radical with 4 to 6 C atoms or a morpholino radical,
R⁸ is a straight-chain or branched-chain alkenyl or alkinyl radical with 2 to 6 carbon atoms, which optionally can be partially or completely fluorinated,
R¹³ is a methyl group or an ethyl group,
R¹⁶ is a fluorine atom in α-position,
R¹⁷ and R^{17'}, in each case independently of one another, are a hydrogen atom and a hydroxy group; or
a hydrogen atom and a group R¹⁸O-, R²⁰SO₂- or OC(=O)R²³ with R¹⁸, R²⁰ and R²³ in each case in the meaning that is indicated under R³ wherein the term "aryl" is a phenyl 1- or 2-naphthyl radical and wherein an "aralkyl" radical is a radical that contains in the ring up to 14 carbon atoms and in the alkyl chain 1 to 8 carbon atoms.

2. Compounds of general formula I according to claim 1, in which R³ is a hydrogen atom.

3. Compounds of general formula I according to claim 1 or 2, in which R⁸ is a vinyl, ethinyl or prop-1-inyl group.

4. Compounds of general formula I according to any one of the claims 1 to 3 in which R¹⁷ and R^{17'} are a hydrogen atom and a hydroxy group atom.

5. Compounds of general formula I according to any one of the claims 1 to 4 in which R⁸ is a vinyl, ethinyl or prop-1-inyl group, R¹⁶ is a fluorine atom, R¹⁷ and R^{17'} are a hydrogen atom and a hydroxy group atom.

6. Compounds of general formula I to any one of the claims 1 to 5, in which R¹⁷ and R^{17'} are a hydrogen atom and a group R¹⁸-O- or R¹⁹SO₂-O- with R¹⁸ and R¹⁹ in each case in the meaning that is indicated under R³.

7. Estratrienes of general formula I according to claim 1 or 2, namely:
8β-vinyl-16α-fluoro-estra-1,3,5(10)-triene-3,17α-diol
8β-vinyl-16α-fluoro-estra-1,3,5(10)-triene-3,17β-diol

8. Pharmaceutical compositions that contain at least a compound according to one of claims 1 to 7 as well as a pharmaceutically compatible vehicle.

9. The compounds of general formula I as defined in any one of the claims 1 to 7 for use as a medicament.

10. Use of the compounds of general formula I as defined in any one of the claims 1 to 7 for the production of a pharmaceutical agents.

11. A compound according to claim 9 for treatment of estrogen-deficiency-induced diseases and conditions in women and in men.

12. A compound according to claim 9 for treatment of peri- and postmenopausal symptoms.

13. A compound according to claim 9 for the in-vitro treatment of female infertility.

14. A compound according to claim 9 for the in-vivo treatment of female infertility.

15. A compound according to claim 9 for the therapy of hormone-deficiency-induced symptoms in ovarian dysfunction that is caused by surgery, medication, etc.

16. A compound according to claim 9 for hormone replacement therapy (HRT).

17. A compound according to claim 11 in combination with selective estrogen receptor modulators (SERM), for example raloxifene.

18. A compound according to claim 9 for prophylaxis and therapy of rheumatoid arthritis multiple sclerosis, and lupus.

19. A compound according to claim 9 for prophylaxis and therapy of inflammatory intestinal diseases and in particular Crohn's disease

20. A compound according to claim 9 for prophylaxis and therapy of inflammatory diseases of the skin and in particular psoriasis.

21. A compound according to claim 9 for prophylaxis and therapy of cardiovascular diseases.

22. A compound according to claim 9 for prophylaxis and therapy of arteriosclerosis, high blood pressure and hypertensive heart disease.

23. A compound according to claim 9 for prevention and treatment of prostate hyperplasia.

24. A compound according to claim 9 in combination with antiestrogens and/or selective estrogen receptor modulators (SERM) for prophylaxis and therapy of prostate hyperplasia.

25. A compound according to claim 9 for treatment of diseases of the immune system.

26. A compound according to claim 9 for the treatment of endometriosis.

27. A compound according to claim 9 for the treatment of cancer of the colon and small intestine.

28. Use of the compounds according to claims 10 for the non-therapeutic stimulation of hair growth.

## Patentansprüche

1. 8β-substituierte Estra-1,3,5(10)-trienderivate der allgemeinen Formel I 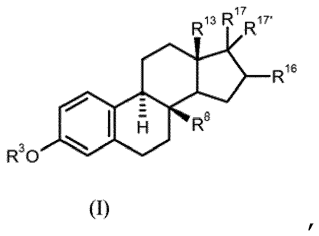 in denen die Reste R³, R⁸, R¹³, R¹⁶ sowie R¹⁷ und R^{17'} unabhängig voneinander die folgenden Bedeutungen haben:
R³ steht für ein Wasserstoffatom oder eine Gruppe R¹⁸, in welcher
R¹⁸ für einen geradkettigen oder verzweigten, gesättigten oder ungesättigten C₁-C₆-Alkylrest, eine Trifluormethylgruppe, einen Aryl-, Heteroaryl- oder Aralkylrest, gegebenenfalls substituiert durch wenigstens einen Rest unabhängig ausgewählt aus Methyl, Ethyl, Trifluormethyl, Pentafluorethyl, Trifluormethylthio, Methoxy, Ethoxy, Nitro, Cyano, Halogen, Hydroxy, Amino, Mono(C₁-C₈-alkyl)- oder Di(C₁-C₈-alkyl)amino, wobei die beiden Alkylgruppen gleich oder verschieden sind, Di(aralkyl)amino, wobei die beiden Aralkylgruppen gleich oder verschieden sind, Carboxyl, Carboxyalkoxy, C₁-C₂₀-Acyl oder C₁-C₂₀-Acyloxy als Substituenten;
einen Acylrest -C(=O)R¹⁹, in welchem R¹⁹ für eine geradkettige oder verzweigte Kette eines C₁-C₁₀-Alkylrests steht, die gesättigt oder an bis zu drei Stellen ungesättigt ist und teilweise oder vollständig halogeniert ist, steht, oder
R¹⁸ für eine Gruppe R²⁰ SO₂ steht, in welcher R²⁰ für eine R²¹R²²N-Gruppe steht, wobei R²¹ und R²² unabhängig voneinander für ein Wasserstoffatom, einen C₁-C₅-Alkylrest, eine Gruppe -C(=O)R²³, in welcher R²³ für einen unsubstituierten oder substituierten, geradkettigen oder verzweigten C₁-C₁₀-Alkylrest steht, welcher gesättigt oder an bis zu drei Stellen ungesättigt ist und teilweise oder vollständig halogeniert ist, eine Cyclopropyl-, Cyclobutyl-, Cyclopentyl-, Cyclohexyl- oder Cycloheptylgruppe, einen C₄-C₁₅-Cycloalkylalkylrest mit 3 bis 7 Kohlenstoffatomen im Cycloalkylteil und mit einem Alkylteil von bis zu 8 Kohlenstoffatomen oder einen Aryl-, Heteroaryl- oder Aralkylrest, gegebenenfalls substituiert durch wenigstens einen Rest unabhängig ausgewählt aus Methyl, Ethyl, Trifluormethyl, Pentafluorethyl, Trifluormethylthio, Methoxy, Ethoxy, Nitro, Cyano, Halogen, Hydroxy, Amino, Mono(C₁-C₈-alkyl)- oder Di(C₁-C₈-alkyl)amino, wobei die beiden Alkylgruppen gleich oder verschieden sind, Di(aralkyl)amino, wobei die beiden Aralkylgruppen gleich oder verschieden sind, Carboxyl, Carboxyalkoxy, C₁-C₂₀-Acyl- oder C₁-C₂₀-Acyloxygruppen als Substituenten; oder zusammen mit dem Stickstoffatom für einen Polymethyleniminorest mit 4 bis 6 Kohlenstoffatomen oder einem Morpholinorest steht,
R⁸ für einen geradkettigen oder verzweigten Alkenyl- oder Alkinylrest mit 2 bis 6 Kohlenstoffatomen, welcher gegebenenfalls teilweise oder vollständig fluoriert sein kann, steht,
R¹³ für eine Methylgruppe oder eine Ethylgruppe steht,
R¹⁶ für ein Fluoratom in der α-Position steht,
R¹⁷ und R^{17'} jeweils unabhängig voneinander für ein Wasserstoffatom und eine Hydroxylgruppe; oder ein Wasserstoffatom und eine Gruppe R¹⁸O-, R²⁰SO₂- oder OC (=O) R²³ stehen, wobei R¹⁸, R²⁰ und R²³ jeweils die unter R³ angegebene Bedeutung haben, wobei der Ausdruck "Aryl" für einen Phenyl-, 1- oder 2-Naphthylrest steht und wobei es sich bei einem "Aralkyl"-Rest um einen Rest handelt, der im Ring bis zu 14 Kohlenstoffatome und in der Alkylkette 1 bis 8 Kohlenstoffatome enthält.

2. Verbindungen der allgemeinen Formel I nach Anspruch 1, in denen R³ für ein Wasserstoffatom steht.

3. Verbindungen der allgemeinen Formel I nach Anspruch 1 oder 2, in denen R⁸ für eine Vinyl-, Ethinyloder Prop-1-inylgruppe steht.

4. Verbindungen der allgemeinen Formel I nach einem der Ansprüche 1 bis 3, in denen R¹⁷ und R^{17'} für ein Wasserstoffatom und eine Hydroxylgruppe stehen.

5. Verbindungen der allgemeinen Formel I nach einem der Ansprüche 1 bis 4, in denen R⁸ für eine Vinyl-, Ethinyl- oder Prop-1-inylgruppe steht, R¹⁶ für ein Fluoratom steht und R¹⁷ und R^{17'} für ein Wasserstoffatom und eine Hydroxylgruppe stehen.

6. Verbindungen der allgemeinen Formel I nach einem der Ansprüche 1 bis 5, in denen R¹⁷ und R^{17'} für ein Wasserstoffatom und eine Gruppe R¹⁸-O- oder R¹⁹SO₂-O-stehen, wobei R¹⁸ und R¹⁹ jeweils die unter R³ angegebene Bedeutung haben.

7. Estratriene der allgemeinen Formel I nach Anspruch 1 oder 2, nämlich:
8β-Vinyl-16α-fluorestra-1,3,5(10)-trien-3,17α-diol
8β-Vinyl-16α-fluorestra-1,3,5(10)-trien-3,17β-diol.

8. Pharmazeutische Zusammensetzung, enthaltend wenigstens eine Verbindung nach einem der Ansprüche 1 bis 7 sowie ein pharmazeutisch unbedenkliches Vehikel.

9. Verbindungen der allgemeinen Formel I nach einem der Ansprüche 1 bis 7 zur Verwendung als Medikament.

10. Verwendung der Verbindungen der allgemeinen Formel I nach einem der Ansprüche 1 bis 7 zur Herstellung eines pharmazeutischen Mittels.

11. Verbindung nach Anspruch 9 zur Behandlung von durch Estrogenmangel induzierten Krankheiten und Leiden bei Frauen und Männern.

12. Verbindung nach Anspruch 9 zur Behandlung von peri- und postmenopausalen Symptomen.

13. Verbindung nach Anspruch 9 zur in-vitro-Behandlung von weiblicher Unfruchtbarkeit.

14. Verbindung nach Anspruch 9 zur in-vivo-Behandlung von weiblicher Unfruchtbarkeit.

15. Verbindung nach Anspruch 9 zur Therapie von durch operative Eingriffe, Medikamente usw. verursachtem Hormonmangel induzierten Symptomen bei einer Eierstockdysfunktion.

16. Verbindung nach Anspruch 9 zur Hormonersatztherapie (Hormone Replacement Therapy, HRT).

17. Verbindung nach Anspruch 11 in Kombination mit selektiven Estrogenrezeptormodulatoren (Selective Estrogen Receptor Modulators, SERM), zum Beispiel Raloxifen.

18. Verbindung nach Anspruch 9 zur Prophylaxe und Therapie von rheumatoider Arthritis, multipler Sklerose und Lupus.

19. Verbindung nach Anspruch 9 zur Prophylaxe und Therapie von entzündlichen Darmerkrankungen und insbesondere Morbus Chron.

20. Verbindung nach Anspruch 9 zur Prophylaxe und Therapie von entzündlichen Hautkrankheiten und insbesondere Psoriasis.

21. Verbindung nach Anspruch 9 zur Prophylaxe und Therapie von Herzkreislauferkrankungen.

22. Verbindung nach Anspruch 9 zur Prophylaxe und Therapie von Arteriosklerose, hohem Blutdruck und hypertensiver Kardiopathie.

23. Verbindung nach Anspruch 9 zur Prävention und Behandlung von Prostatahyperplasie.

24. Verbindung nach Anspruch 9 in Kombination mit Antiestrogenen und/oder selektiven Estrogenrezeptormodulatoren (SERM) zur Prophylaxe und Therapie von Prostatahyperplasie.

25. Verbindung nach Anspruch 9 zur Behandlung von Erkrankungen des Immunsystems.

26. Verbindung nach Anspruch 9 zur Behandlung von Endometriose.

27. Verbindung nach Anspruch 9 zur Behandlung von Kolonkrebs und Dünndarmkrebs.

28. Verwendung der Verbindungen nach Anspruch 10 zur nichttherapeutischen Stimulierung des Haarwachstums.

## Revendications

1. Dérivés d'estra-1,3,5(10)-triène 8β-substitués de formule générale I 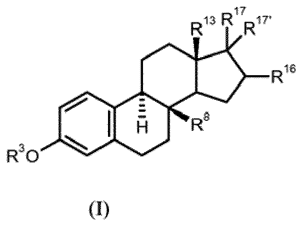 dans laquelle les radicaux R³, R⁸, R¹³, R¹⁶ ainsi que R¹⁷ et R^{17'}, indépendamment l'un de l'autre, revêtent la signification suivante :
R³ désigne un atome d'hydrogène ou un groupement R¹⁸, où
R¹⁸ désigne un radical C₁-C₆-alkyle à chaîne linéaire ou chaîne ramifiée, saturé ou insaturé, un groupement trifluorométhyle, un radical aryle, hétéroaryle ou aralkyle, éventuellement substitué par au moins un radical choisi indépendamment parmi un groupement méthyle, éthyle, trifluorométhyle, pentafluoroéthyle, trifluorométhylthio, méthoxy, éthoxy, nitro, cyano, halogéno, hydroxy, amino, mono(C₁-C₈-alkyl) ou di(C₁-C₈-alkyl) amino où les deux groupements alkyle sont identiques ou différents, di(aralkyl)amino où les deux groupements aralkyle sont identiques ou différents, carboxyle, carboxyalcoxy, C₁-C₂₀-acyle ou C₁-C₂₀-acyloxy comme substituants ;
un radical acyle -C(=O)R¹⁹, où R¹⁹ est une chaîne linéaire ou une chaîne ramifiée d'un radical C₁-C₁₀-alkyle qui est saturé ou insaturé jusqu'en trois endroits et est partiellement ou complètement halogéné, ou
R¹⁸ désigne un groupement R²⁰SO₂, où
R²⁰ est un groupement R²¹R²²N, où R²¹ et R²², indépendamment l'un de l'autre, désignent un atome d'hydrogène, un radical C₁-C₅-alkyle, un groupement -C(=O)R²³, où R²³ désigne un radical C₁-C₁₀-alkyle non substitué ou substitué, à chaîne linéaire ou chaîne ramifiée, qui est saturé ou insaturé jusqu'en trois endroits et est partiellement ou complètement halogéné, un groupement cyclopropyle, cyclobutyle, cyclopentyle, cyclohexyle ou cycloheptyle, un radical C₄-C₁₅-cycloalkylalkyle ayant de 3 à 7 atomes de carbone dans la portion cycloalkyle et ayant une portion alkyle ayant jusqu'à 8 atomes de carbone ou un radical aryle, hétéroaryle ou aralkyle éventuellement substitué par au moins un radical choisi indépendamment parmi un groupement méthyle, éthyle, trifluorométhyle, pentafluoroéthyle, trifluorométhylthio, méthoxy, éthoxy, nitro, cyano, halogéno, hydroxy, amino, mono(C₁-C₈-alkyl) ou di(C₁-C₈-alkyl) amino où les deux groupements alkyle sont identiques ou différents, di(aralkyl)amino où les deux groupements aralkyle sont identiques ou différents, carboxyle, carboxyalcoxy, C₁-C₂₀-acyle ou C₁-C₂₀-acyloxy comme substituants ; ou,
conjointement avec l'atome de N, un radical polyméthylèneimino ayant de 4 à 6 atomes de C ou un radical morpholino,
R⁸ est un radical alcényle ou alcynyle à chaîne linéaire ou chaîne ramifiée ayant de 2 à 6 atomes de carbone, qui peut éventuellement être partiellement ou complètement fluoré,
R¹³ est un groupement méthyle ou un groupement éthyle,
R¹⁶ est un atome de fluor en position α,
R¹⁷ et R^{17'} sont dans chaque cas, indépendamment l'un de l'autre, un atome d'hydrogène et un groupement hydroxy ; ou
un atome d'hydrogène et un groupement R¹⁸O-, R²⁰SO₂- ou OC(=O)R²³ ; R¹⁸, R²⁰ et R²³ revêtant dans chaque cas la signification indiquée pour R³,
où par le terme « aryle » on entend un radical phényle, 1- ou 2-naphtyle et où un radical « aralkyle » est un radical qui contient jusqu'à 14 atomes de carbone dans le cycle et de 1 à 8 atomes de carbone dans la chaîne alkyle.

2. Composés de formule générale I selon la revendication 1, dans lesquels R³ est un atome d'hydrogène.

3. Composés de formule générale I selon la revendication 1 ou 2, dans lesquels R⁸ est un groupement vinyle, éthinyle ou prop-1-inyle.

4. Composés de formule générale I selon l'une quelconque des revendications 1 à 3, dans lesquels R¹⁷ et R^{17'} sont un atome d'hydrogène et un groupement hydroxy.

5. Composés de formule générale I selon l'une quelconque des revendications 1 à 4, dans lesquels R⁸ est un groupement vinyle, éthinyle ou prop-1-inyle, R¹⁶ est un atome de fluor, R¹⁷ et R^{17'} sont un atome d'hydrogène et un groupement hydroxy.

6. Composés de formule générale I selon l'une quelconque des revendications 1 à 5, dans lesquels R¹⁷ et R^{17'} sont un atome d'hydrogène et un groupement R¹⁸-O-ou R¹⁹SO₂-O-, R¹⁸ et R¹⁹ revêtant dans chaque cas la signification indiquée pour R³.

7. Estratriènes de formule générale I selon la revendication 1 ou 2, à savoir :
le 8β-vinyl-16α-fluoro-estra-1,3,5(10)-triène-3,17α-diol,
le 8β-vinyl-16α-fluoro-estra-1,3,5(10)-triène-3,17β-diol.

8. Compositions pharmaceutiques contenant au moins un composé selon l'une quelconque des revendications 1 à 7, ainsi qu'un véhicule pharmaceutiquement compatible.

9. Composés de formule générale I telle que définie selon l'une quelconque des revendications 1 à 7, pour une utilisation comme médicament.

10. Utilisation des composés de formule générale I telle que définie selon l'une quelconque des revendications 1 à 7, pour la production d'un agent pharmaceutique.

11. Composé selon la revendication 9, pour le traitement de maladies et de conditions induites par une déficience en oestrogènes chez la femme et chez l'homme.

12. Composé selon la revendication 9, destiné au traitement de symptômes de la péri- et post-ménopause.

13. Composé selon la revendication 9, destiné au traitement in *vitro* de l'infertilité chez la femme.

14. Composé selon la revendication 9, destiné au traitement in *vivo* de l'infertilité chez la femme.

15. Composé selon la revendication 9, destiné à la thérapie de symptômes induits par une déficience hormonale dans le cas d'un dysfonctionnement ovarien provoqué par une opération chirurgicale, un médicament, etc.

16. Composé selon la revendication 9, destiné à une hormonothérapie substitutive (HTS).

17. Composé selon la revendication 11, en combinaison avec des modulateurs sélectifs des récepteurs d'oestrogènes (SERM), par exemple le raloxifène.

18. Composé selon la revendication 9, destiné à la prophylaxie et à la thérapie de la polyarthrite rhumatoïde, de la sclérose en plaques et du lupus.

19. Composé selon la revendication 9, destiné à la prophylaxie et à la thérapie de maladies intestinales inflammatoires et en particulier de la maladie de Crohn.

20. Composé selon la revendication 9, destiné à la prophylaxie et à la thérapie de maladies inflammatoires de la peau et en particulier du psoriasis.

21. Composé selon la revendication 9, destiné à la prophylaxie et à la thérapie de maladies cardiovasculaires.

22. Composé selon la revendication 9, destiné à la prophylaxie et à la thérapie de l'artériosclérose, d'une pression artérielle élevée et d'une maladie cardiaque hypertensive.

23. Composé selon la revendication 9, destiné à la prévention et au traitement de l'hyperplasie de la prostate.

24. Composé selon la revendication 9, en combinaison avec des anti-estrogènes et/ou des modulateurs sélectifs des récepteurs d'oestrogènes (SERM), destiné à la prophylaxie et à la thérapie de l'hyperplasie de la prostate.

25. Composé selon la revendication 9, destiné au traitement de maladies du système immunitaire.

26. Composé selon la revendication 9, destiné au traitement de l'endométriose.

27. Composé selon la revendication 9, destiné au traitement du cancer du côlon et de l'intestin grêle.

28. Utilisation des composés selon la revendication 10, pour une stimulation non thérapeutique de la croissance capillaire.
